# EUROPÄISCHE PATENTANMELDUNG

(11) **EP 0 607 558 A1**
(43) Veröffentlichungstag der Anmeldung: **27.07.1994**
(21) Anmeldenummer: 93119924.4
(22) Anmeldetag: 10.12.1993
(51) Int. Cl.: G01N 22/00, B08B 9/46

(54) **Verfahren zur Ermittlung von flüssigen Substanzen in einem Behälter**

(30) Priorität: 18.01.1993 CH 133/93; 18.11.1993 CH 3441/93
(71) Anmelder: ELPATRONIC AG, CH-6303 Zug (CH)
(72) Erfinder: Gernet, Pierre, CH-5400 Ennetbaden (CH); Jungo, Daniel, CH-8116 Würenlos (CH)

(57) **Zusammenfassung**

Der Behälter wird mit Mikrowellen beaufschlagt. Aus der Absorption der Mikrowellenenergie durch die im Behälter befindliche Substanz wird auf deren Menge und Art geschlossen. Insbesondere vorteilhaft ist das Verfahren zur Ermittlung von Restflüssigkeit in einer gewaschenen Mehrwegflasche vor deren erneuter Befüllung.

## Beschreibung

Die Erfindung betrifft ein verfahren zur Ermittlung von flüssigen Substanzen in einem Behälter.

Insbesondere bei Mehrweg-Getränkeflaschen (z.B. PET-Flaschen) ist es üblich, nach dem waschen und vor dem erneuten Befüllen derselben eine Prüfung auf das Vorhandensein von Restflüssigkeit in der Flasche durchzuführen. Dabei handelt es sich um Restmengen von Wasser oder Waschlauge oder auch um ölige Rückstände.

Bestehende Restlaugensysteme können Restlaugen bis 1 ml 0,5% NAOH und bis hinunter zu 6 ml H₂O feststellen. Diese messen im Prinzip die Flasche als Dielektrikum in einem HF Feld mit 30 Mhz. Dieses Messystem besitzt jedoch keine Empfindlichkeit für ölige Reste. Auch die Art der Restflüssigkeit kann auf diese Weise nicht bestimmt werden.

Der Erfindung liegt deshalb die Aufgabe zugrunde, ein Verfahren zur Ermittlung von Restflüssigkeiten zu schaffen, welches rasch und kostengünstig auch ölige Rückstände erfassen kann, und welches grundsätzlich die Unterscheidung verschiedener Arten von Restflüssigkeiten erlaubt.

Dies wird mit den kennzeichnenden Merkmalen des Anspruches 1 erreicht.

Durch die Beaufschlagung des Behälters mit Mikrowellen im beanspruchten Bereich kann das Vorhandensein derselben sehr genau bestimmt werden. Durch die geeignete Wahl der Frequenz lässt sich grundsätzlich jede Art von Restflüssigkeit ermitteln. Die geeignete Frequenz lässt dabei andererseits Rückschlüsse auf die Art der Substanz zu.

Vorzugsweise wird nicht nur mit einer fixen Frequenz gearbeitet, sondern die Frequenz wird variiert, z.B. als Abfolge diskreter Frequenzen oder durchlaufend über einen Bereich.

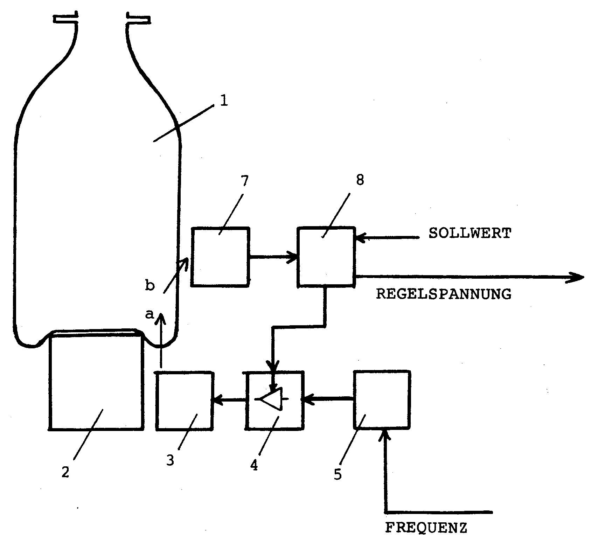

Im folgenden werden Ausführungsbeispiele der Erfindung anhand der Figur näher erläutert. Diese zeigt schematisch eine Messeinrichtung für Restflüssigkeit in einer Flasche.

Gezeigt ist in der Figur eine Flasche 1 als Beispiel für einen Behälter. Es handelt sich dabei vorzugsweise um eine PET-Kunststoff-Mehrwegflasche.

Die Flasche 1, welche vorzugsweise eine von einer Mehrzahl von Flaschen ist, die auf einer Fördereinrichtung (z.B. einer Karussellfördereinrichtung oder einer Linienfördereinrichtung) gefördert werden, steht mit ihrem Boden auf einer Halterung 2 auf, welche den Bodenrandbereich der Flasche 1 freilässt. Im Bodenrandbereich verteilt sich die allfällig nach dem Waschen der Flasche vorhandene Restflüssigkeitsmenge, welche aus Wasser, Waschlauge NAOH 0,5% oder öligen Flüssigkeiten bestehen kann.

Von einem Sender 3, 4, 5 wird ein hochfrequentes elektro-magnetisches Feld erzeugt, was durch die Pfeile a, b dargestellt werden soll. Dieses Feld durchdringt den Bodenbereich der Flasche und wird von einem Empfänger 7 empfangen. Eine bevorzugte Frequenz der Strahlung ist etwa 2,4 GHz. Diese Frequenz ist die typische Resonanzfrequenz von Wassermolekülen. Durch das Feld werden die Moleküle in der Flasche in Schwingung versetzt, wodurch eine Erwärmung der Flüssigkeit durch die Molekularreibung erfolgt. Die Absorbtion der HF-Energie wird erkannt und kann auch quantifiziert werden, wodurch direkt die Menge der Restflüssigkeit ermittelt werden kann.

Wird nun während des Messvorganges die Frequenz mit einem Wobbler verändert, z.B. im Bereich von 2 bis 2,5 GHz, besteht sogar die Möglichkeit, gewisse Medien zu identifizieren. Jede der Flüssigkeiten hat eine für sie typische Resonanzfrequenz. Durch diese Messung entsteht dann ein Spektrum der Flüssigkeitsmengen, welches ausgewertet werden kann. Man kann damit Grenzwerte der Quantität für verschiedene Stoffe untersuchen.

Anstelle eines Wobblers kann auch ein umschaltbarer Oszilator verwendet werden, der die Resonanzfrequenzen von den wichtigsten zu untersuchenden Flüssigkeiten generiert. Man erhält dann ein diskretes Spektrum mit Peaks, welches aber schneller ausgewertet werden kann. Es können auch mehrere Sender vorgesehen sein, welche auch voneinander verschiedene Empfindlichkeiten aufweisen können.

Der Sender ist in der Figur mit einem einstellbaren Oszillator 5, einem einstellbaren Verstärker 4 und einer eigentlichen Sendeschaltung mit Antenne 3 gezeigt.

Der Oszillator 5 erzeugt eine hochfrequente Strahlung, die in Richtung des Empfängers gesendet wird. Die am Empfänger 7 empfangene Energie wird gemessen und mit einem Sollwert verglichen. Dann regelt ein Regler 8 den Verstärker 4 so, dass die empfangene Energie diesem Sollwert entspricht. Diese Regelspannung ist der Messausgang.

Wird nun durch eine Restflüssigkeit Energie aus diesem Regelkreis abgezogen, kompensiert der Regler dies. Das Mass der absorbierten Energie entspricht der Menge der Flüssigkeit mit der typischen Resonanzfrequenz.

Die Flasche selbst absorbiert nur unwesentlich auf diesen Frequenzen. Dieser Anteil ist dazu konstant.

Durch das erfindungsgemässe Verfahren können kleinere Restmengen detektiert werden als bisher. Zudem können auch andere Grundstoffe als Wasser und Natronlauge (wie bisher) erkannt werden. Es können insbesondere auch ölige oder alkoholische Flüssigkeiten und andere erkannt und quantifiziert werden.

Das Verfahren bietet sich daher allgemein zur Ermittlung von flüssigen Substanzen an, die in einem Behälter befindlich sind. Auch die Reflexion, z.B. durch ein Metallteil im Behälter, kann detektiert werden.

## Patentansprüche

1. Verfahren zur Ermittlung von flüssigen Substanzen in einem nicht metallischen Behälter, insbesondere in einer Kunststoff-Flasche, dadurch gekennzeichnet, dass der Behälter mindestens teilweise einem hochfrequenten elektro-magnetischen Feld im Bereich von 2 bis 3 Gigahertz ausgesetzt wird, und dass die Beeinflussung des Feldes durch die Schwingungsanregung der Flüssigkeitsmoleküle detektiert wird.

2. Verfahren nach Anspruch 1, dadurch gekennzeichnet, dass während der Ermittlung die Frequenz des Feldes variiert wird, um verschiedene Flüssigkeitssubstanzen zu unterscheiden.

3. Verfahren nach Anspruch 1 oder 2, dadurch gekennzeichnet, dass der Behälter eine zur Reinigung und Neubefüllung rücklaufende Kunststoff-Mehrwegflasche, insbesondere eine PET-Flasche, ist, deren Restflüssigkeitsinhalt ermittelt wird.

4. Verfahren nach Anspruch 3, dadurch gekennzeichnet, dass der Bodenbereich der stehend angeordneten Flasche dem Feld ausgesetzt wird.

5. Verfahren nach einem der Ansprüche 1 bis 4, dadurch gekennzeichnet, dass von einer Mehrzahl von Behältern jeweils einer in den Sende/ Empfangsbereich eines Mikrowellensenders (3, 4, 5) und eines Mikrowellenempfängers (7) gefördert wird, derart, dass der Empfänger überwiegend Sendesignale empfängt, welche durch den Behälter hindurch getreten sind.

6. Verfahren nach Anspruch 5, dadurch gekennzeichnet, dass der Sender jeweils nachgeregelt wird, bis das Empfangssignal einen vorbestimmten Wert erreicht, und dass das Regelsignal als Mass für die Restflüssigkeit ausgewertet wird.

7. Vorrichtung zur Durchführung des Verfahrens nach Anspruch 5, gekennzeichnet durch eine Behälterfördereinrichtung und eine Sende/ Empfangseinrichtung für Mikrowellen.

8. Vorrichtung nach Anspruch 7, dadurch gekennzeichnet, dass die Behälterfördereinrichtung eine Flaschenfördereinrichtung ist.
